# NEUE EUROPÄISCHE PATENTSCHRIFT

(11) **EP 0 857 719 B2**
(45) Veröffentlichungstag und Bekanntmachung des Hinweises auf die Entscheidung über den Einspruch: **26.07.2006**
(45) Hinweis auf die Patenterteilung: 17.04.2002
(21) Anmeldenummer: 97123045.3
(22) Anmeldetag: 31.12.1997
(51) Int. Cl.: C07D 211/58

(54) **Kontinuierliches Verfahren zur Herstellung von 4-Aminopiperidinen**
Continuous process for the preparation of 4-aminopiperidines
Procédé continu pour la préparation de 4-aminopipéridines

(30) Priorität: 06.02.1997 DE 19704460
(43) Veröffentlichungstag der Anmeldung: 12.08.1998
(73) Patentinhaber: Degussa AG, 40474 Düsseldorf (DE)
(72) Erfinder: Jegelka, Udo, Dr., 45657 Recklinghausen (DE); Kreilkamp, Günter, 45770 Marl (DE)

(56) Entgegenhaltungen:
- EP-A- 0 013 665
- EP-A- 0 033 529
- EP-A- 0 202 001
- EP-A- 0 208 445
- EP-A- 0 302 020
- EP-A- 0 354 184
- DE-A- 2 621 870
- DE-A- 3 007 996
- Ullmann's Encyclopedia of Industrial Chemistry, 5. Auflage, Bd. B4, S. 222-223, VCH Weinheim, 1992
- P. Rylander, Catalysis of Organic Reactions, 1988, S. 178-180
- Ullmann's Encyclopedia of Industrial Chemistry, 5. Auflage, Bd. A2, S. 4-5, VCH Weinheim, 1985
- P. Rylander, Catalytic Hydrogenation in Organic Syntheses, 1979, S. 6-7
- J. Falbe und U. Hasserodt (Herausgeber), "Katalysatoren, Tenside und Mineraloeladditive, S. 45-48, Thieme Verlag, 1978

## Beschreibung

Die Erfindung betrifft ein kontinuierliches Verfahren zur Herstellung von N-monosubstituierten 4-Aminopiperidinen, welche in erster Linie Ausgangsstoffe zur Herstellung von sterisch gehinderten Aminen sind, die als Stabilisatoren für synthetische Polymere dienen und als H.A.L.S. (Hindered Amine Light Stabilizers) bezeichnet werden.

Die Herstellung von N-monosubstituierten 4-Aminopiperidinen aus den entsprechenden 4-Oxopiperidinen (oder 4-Piperidonen), primären Aminen und Wasserstoff unter Hydrierbedingungen, also durch aminierende Hydrierung oder reduktive Aminierung, ist seit langem bekannt und wurde u.a. in zahlreichen Patentschriften beschrieben. Bisher wurde dieses Verfahren in einer Stufe oder überwiegend in zwei Stufen und, was für die vorliegende Erfindung von Bedeutung ist, stets absatzweise durchgeführt. Ein kontinuierlich arbeitendes Verfahren wäre in hohem Maße erwünscht. Trotz zahlreicher Bemühungen zahlreicher Forschungs- und Entwicklungsgruppen ist es jedoch bisher nicht gelungen, ein brauchbares kontinuierliches Verfahren zu entwickeln.

Bei der zweistufigen absatzweisen Arbeitsweise wird in der ersten Stufe die Schiffsche Base erzeugt, die dann nach Entfernung des Reaktionswassers in der zweiten Stufe katalytisch zum N-monosubstituierten, also sekundären Amin hydriert wird. Bei der einstufigen Arbeitsweise laufen beide Reaktionen ohne Abtrennung des Reaktionswassers nebeneinander ab.

In EP 0 354 184 wird die ein- oder zweistufige reduktive Aminierung von Triacetonaminderivaten mit Diaminen in Gegenwart katalytischer Mengen von Platin, Palladium oder Nickel beschrieben. Bei der zweistufigen Variante wird Wasser azeotrop abdestilliert und so die Bildung der Schiffschen Base erzwungen. Die einstufige Arbeitsweise läßt sich nur in Gegenwart von Säuren als Co-katalysator durchführen.

Die DE 3 007 996 beschreibt zwar die einstufige reduktive Aminierung von 4-Piperidonen mit Alkylaminen oder Alkylendiaminen in einem inerten Lösemittel mit Raney-Nickel oder Raney-Cobalt als Katalysator. Die zweistufige Arbeitsweise mit azeotroper Entfernung des Reaktionswassers, zweckmäßig unter Mitverwendung eines Ammoniumsalzes als Co-katalysator, wird jedoch als vorteilhaft bezeichnet.

Bei dem zweistufigen Verfahren nach EP 0 033 663 werden in den beiden Stufen unterschiedliche Lösemittel verwendet. So wird in der ersten Stufe Heptan (zugleich als Schleppmittel für das Reaktionswasser) und in der Hydrierstufe Isopropanol benutzt.

Natürlich wäre es erwünscht, wenn man die N-monosubstituierten 4-Aminopiperidine mit gleichem Erfolg oder gar besser in nur einer Stufe herstellen könnte. So gibt es denn auch eine Reihe von Patentschriften, die speziell auf eine solche Arbeitsweise gerichtet sind. In EP 0 013 665 und in EP 0 061 785 wird die einstufige reduktive Aminierung von Triacetonaminderivaten mit Di- oder höheren Polyaminen in Gegenwart von Methanol mit Platin auf Kohle als Katalysator beschrieben. Dabei muß allerdings konzentrierte Schwefelsäure als Co-katalysator mitverwendetwerden.

In DE 26 21 870 wird die reduktive Aminierung von Alkylpiperidonen mit Methylamin oder Ammoniak in Methanol unter Verwendung von 15 % Raney-Nickel als Katalysator beschrieben Die EP 0 208 455 offenbart die einstufige reduktive Aminierung von Piperidonen mit Alkylaminen oder Alkylendiaminen in einem Alkohol oder Glykol und in Gegenwart von Cobalt-, Nickel- oder Platinkatalysatoren. Zur Senkung der Entflammbarkeit werden als Lösemittel Alkohol-Wasser-Gemische mit einem Wasseranteil von mindestens 10 Vol.-% verwendet. Ein analoges Verfahren mit Palladium als Katalysator beschreibt die EP 0 202 001.

Die EP 0 081 688 und 0 045 048 sowie JP 76009486 beschreiben die einstufige reduktive Aminierung von Piperidonen mit Dialkyaminen oder Trialkylaminen in Methanol und in Gegenwart von Platin auf Kohle als Katalysator. In EP 0 302 020 wird die einstufige reduktive Aminierung von Piperidonen mit Alkylaminen ohne Lösemittel offenbart, wobei bis zu 10 Gew.-% Wasser im Reaktionsgemisch verbleiben darf.

In jüngster Zeit geht die Entwicklung wieder zu den zweistufigen Verfahren hin, weil es bisher nicht gelungen ist, die an sich bevorzugten einstufigen Verfahren voll befriedigend zu entwickeln. So wird in EP 0 508 940 die zweistufige reduktive Aminierung von Piperidonen mit Diaminen beschrieben. In der ersten Stufe wird in Abwesenheit eines Lösemittels durch Abdestillieren des Reaktionswassers die Schiffsche Base erzeugt. In der zweiten Stufe wird die wasserfreie Schiffsche Base an Palladium, Platin oder Raney-Nickel hydriert. Als Vorteil des Verfahrens wird angegeben, daß infolge der Zweistufigkeit eine Schiffsche Base von hoher Reinheit erzeugt wird, wodurch die nachfolgende Hydrierung auch bei höheren Temperaturen selektiver und schneller als bisher verläuft.

Die ausnahmslos absatzweise arbeitenden Verfahren nach dem Stand der Technik haben eine Reihe von Nachteilen. Zum einen ist das ständige An- und Abfahren der einzelnen Ansätze mit vergleichsweise hohem Personalaufwand verbunden und führt zudem zu geringen Raum-Zeit-Ausbeuten und erhöhtem Energieverbrauch. Die meisten bekannten Verfahren arbeiten mit entzündlichen Lösemitteln, was einerseits die Raum-Zeit-Ausbeuten weiter herabsetzt, zum anderen höheren Aufwand für Brandschutzmaßnahmen bedingt und zudem auch noch höhere Materialkosten verursacht.

Weiterhin sind die feinteiligen Hydrierkatalysatoren in der Regel pyrophor und daher selbst bei Abwesenheit von brennbaren Lösemitteln nur unter Vorsichtsmaßnahmen zu handhaben, sowohl bei der Erstbeschickung, als auch bei späteren Ansätzen. Femer sind solche Katalysatoren, wie in EP 0 508 940 festgestellt wird, gegen Katalysatorgifte empfindlich und sind nicht in einfacher Weise regenerierbar. Edelmetallkatalysatoren müssen schon aus Kostengründen wieder aufgearbeitet werden. Die Entsorgung von Nickel- und Kobaltkatalysatoren ist wegen ihrer toxikologischen Eigenschaften problematisch.

Der Umsatz der Edukte und die Selektivität der Bildung des gewünschten N-monosubstituierten 4-Aminopiperidins lassen insbesondere bei der einstufigen Arbeitsweise, die an sich aus verfahrenstechnischen Gründen zu bevorzugen wäre, zu wünschen übrig. Dies ist verständlich, weil bei der einstufigen Arbeitsweise eine Hydrierung der N-substituierten 4-Aminopiperidone zu den entsprechenden 4-Aminopiperidolen stattfinden kann, die bei derzweistufigen Arbeitsweise stark reduziert ist. und zudem weitere Nebenprodukte entstehen. Die lösemittelfreien rohen Reaktionsgemische enthalten jedenfalls selten mehr als 90 Gew.-% des Zielprodukts, dessen Anteil nur durch Mitverwendung eines Co-katalysators, der dann allerdings wieder abgetrennt werden muß, auf 92 bis 93 Gew.-% gesteigert werden kann Das sind Gehalte, die sich mit dem aufwendigeren zweistufigen Verfahren auch ohne Co-katalysator dadurch erreichen lassen, daß man in der ersten Stufe das Reaktionswasser abtrennt.

Wegen der relativ niedrigen Gehalte der rohen Reaktionsgemische an dem Zielprodukt ist es schwierig, ein hinreichend reines N-monosubstituiertes 4-Aminopiperidin herzustellen. Nach DE 41 20 550 erhält man in einem einstufigen, absatzweise arbeitenden Verfahren ein rohes Reaktionsgemisch mit 93 Gew.-% Zielprodukt, 0,8 Gew.-% Eduktketon und einem APHA-Wert (in 10 gewichtsprozentiger Toluollösung) von 400 und daraus durch eine herkömmliche Destillation (Kp. 180 bis 190°C; etwa 1 mbar) einen hellen Feststoff mit >99 Gew.-% Zielprodukt, 100 ppm Eduktketon und einem APHA-Wert von 100. Das Produkt ist jedoch nicht lagerstabil, sondern zeigt nach 3 Monaten bei 60°C einen APHA-Wert von >1.000. Produkte mit ausreichender Lagerstabilitat erhält man nur durch eine absatzweise Kristallisation aus einem Keton als Lösemittel. Die analytischen Daten der Kristalle unterscheiden sich zwar nicht von denen des Destillats. Der APHA-Wert beträgt aber nur ≤30 und steigt nach 3 Monaten bei 60°C nur auf ≤100 an. Es liegt auf der Hand, daß dieser zusätzliche Reinigungsschritt aufwendig und unerwünscht ist.

Als kontinuierliches Verfahren zur Herstellung von 4-Alkylaminopiperidinen ist lediglich die reduktive Alkylierung von 4-Aminopiperidinen mit den entsprechenden Alkanolen nach EP 0 128 285 bekannt. Das Verfahren arbeitet mit hohen Aminüberschüssen, und es sind nur etwa 10 Gew.-% des gewünschten Produktes im Reaktionsaustrag enthalten, so daß man zur Steigerung des Umsatzgrades das nicht umgesetzte Edukt in aufwendigen Zusatzschritten abtrennen und zurückführen muß.

Es wurde nun gefunden, daß sich N-monosubstituierte 4-Aminopiperidine vorteilhaft durch kontinuierliche, einstufige Umsetzung von 4-Oxopiperidinen mit einem primären Amin und Wasserstoff unter Hydrierungsbedingungen vorteilhaft herstellen lassen.

Das Verfahren nach der Erfindung ist mit einer Reihe von Vorteilen verbunden, die die Verfahren nach dem Stand der Technik in dieser Kombination nicht aufweisen:
- Das aufwendige An- und Abfahren einzelner Ansätze entfällt.
- Die Raum-Zeit-Ausbeuten sind dadurch so sehr verbessert, daß für die LHSV (liquid hourly space velocity) Werte von bis zu 1 h⁻¹ erreicht werden.
- Die vorzugsweise verwendeten Trägerkatalysatoren mit Metallen der VIII. Gruppe des Periodensystems können so eingestellt werden, daß sie nicht pyrophor und daher gut handhabbar sind.
- Die Trägerkatalysatoren sind flexibel, und für die verschiedensten Eduktsysteme (4-Oxopiperidin/pimäres Amin) hervorragend geeignet.
- Die Trägerkatalysatoren haben sehr lange Standzeiten, so daß die Kosten für Beschaffung, Ein- und Ausbau, Entsorgung oder Aufarbeitung erheblich vermindert werden.
- Lösemittelverluste und Umweltbelastungen durch Lösemittel, wie sie mit dem Recycling der Katalysatoren bei absatzweise arbeitenden Verfahren verbunden sind, werden vermieden.
- Die Trägerkatalysatoren und damit das Verfahren sind also zugleich ökonomisch und ökologisch vorteilhaft.
- Das Verfahren zeichnet sich im Vergleich mit bisher bekannten Verfahren durch höhere Umsatzgrade aus, die >99% betragen können.
- Infolge der vergleichsweise geringeren thermischen Belastung werden Neben- und Zersetzungsreaktionen zurückgedrängt, so daß eine höhere Selektivität resultiert, die ebenfalls >99%, bezogen auf das 4-Oxopiperidin, betragen kann.
- Diese günstigen Ergebnisse werden in einem kontinuierlichen Verfahren mit nur einer Reaktionsstufe ohne vorherige gesonderte Herstellung der Schiffschen Base, ohne Co-katalysator und ohne Lösemitteleinsatz erzielt.
- Überraschenderweise lassen sich die rohen Reaktionsgemische durch übliche Destillation und ohne aufwendige Kristallisation zu lagerstabilen Produkten reinigen.

N-monosubstituierte 4-Aminopiperidine der allgemeinen Formel 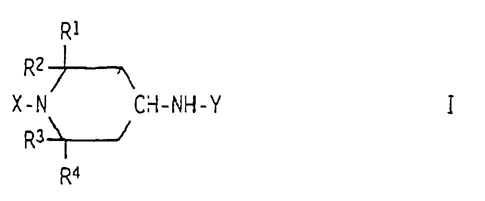 erhält man, wenn man ein 4-Oxopiperidin der allgemeinen Formel 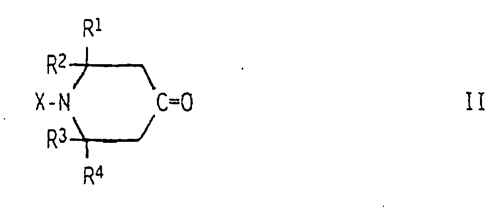 kontinuierlich mit einem primären Amin der Formel

Y-NH₂ III

und Wasserstoff unter Hydrierbedingungen umsetzt.

N-monosubstituierte 4-Aminopiperidine der Formel 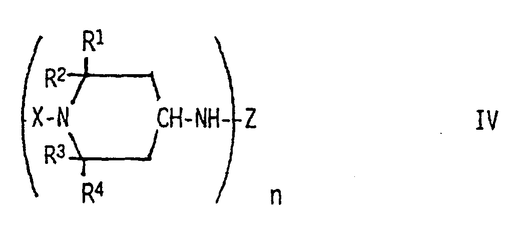 erhält man, wenn man ein 4-Oxopiperidin der Formel 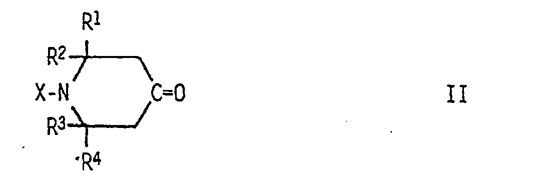 kontinuierlich mit einem Polyamin der Formel

Z-(NH₂), V

als primärem Amin und mit Wasserstoff unter Hydrierbedingungen umsetzt, wobei man die Edukte über einen fest angeordneten Trägerkatalysator führt, der ein oder mehrere Metalle der VIII. Nebengruppe des Periodensystems enthält und man die Reaktion ohne Lösemitteleinsatz bei 80 bis 200°C durchführt. In den Formeln I bis V
- können R¹, R², R³ und R⁴ gleich oder verschieden sein und bedeuten Wasserstoff oder einen Kohlenwasserstoffrest mit 1 bis 4 Kohlenstoffatomen, vorteilhaft einen Methylrest:

- steht X für Wasserstoff; eine Hydroxylgruppe; einen Kohlenwasserstoffrest mit bis zu 18 Kohlenstoffatomen, vorteilhaft einen Alkylrest mit 1 bis 12 Kohlenstoffatomen, einen Cycloalkylrest mit 3 bis 12 Kohlenstoffatomen, einen Aralkylrest mit 7 bis 10 Kohlenstoffatomen oder einen Alkarylrest mit 7 bis 10 Kohlenstoffatomen: einen Acylrest mit 2 bis 10 Kohlenstoffatomen; für eine gegebenenfalls N,N-dialkylierte Amino- oder Aminoalkylgruppe mit bis zu 12 Kohlenstoffatomen; oder für einen endständig alkylierten Alkylenrest der Formel -(CH₂)ₘ-R⁵, in der R⁵ eine Hydroxylgruppe oder einen Alkoxyrest mit 1 bis 6 Kohlenstoffatomen und m eine ganze Zahl von 1 bis 6 bedeutet;
- bedeutet Y einen einwertigen Kohlenwasserstoffrest mit 1 bis 12 Kohlenstoffatomen, der gegebenenfalls Etherbrücken, (N-Alkyl)-iminobrucken und/oder Hydroxylgruppen enthält und/oder arylsubstftuiert ist:

- bedeutet Z einen n-wertigen Kohlenwasserstoffrest mit 2 bis 12 Kohlenstoffatomen, der gegebenenfalls Etherbrucken, (N-Alkyl)-iminobrucken und/oder Hydroxylgruppen enthält und/oder arylsubstituiert ist;
- steht n für eine ganze Zahl von 2 bis 4.

Die Edukte II sind bekannte Stoffe, die z.T. in kommerziellen Mengen erhältlich sind. Als Beispiele seien 4-Oxopiperidin, 2,6-Dimethyl-4-oxopiperidin, 2,6-Diethyl-4-oxopiperidin, 2,2,6,6-Tetramethyl-4-oxopiperidin, 1-Hydroxy-2,2,6,6-tetramethyl-4-oxopiperidin, 1-Methyl-2,2,6,6-tetramethyl-4-oxopiperidin, 1-n-Hexyl-2,2,6,6-tetramethyl-4-oxopiperidin, 1-Acetyl-2,6-dimethyl-4-oxopiperidin, 1-Dimethylamino-2,2,6,6-tetramethyl-4-oxopiperidin und 1-Dimethylaminoethyl-2,2,6,6-tetramethyl-4-oxopiperidin genannt.

Geeignete primäre Amine III sind u.a. Methylamin, Ethylamin, n-Butylamin, Isooctylamin, Stearylamin, Cyclohexylamin, Anilin, Toluidin, Phenylethylamin, 3-Methoxypropylamin, Ethoxyethylamin, Ethoxyethoxyethylamin, 3-Aminopropanol, 2-Ethylaminoethylamin, 2-Dimethylaminoethylamin und Aminoethylmethylethylamin.

Geeignete Polyamine V sind z.B. Ethylendiamin, Butylendiamin. Hexamethylendiamin. 1,12-Diaminododecan, 1,5-Diamino-3-oxa-pentan. 1,5-Diamino-3-methyl-3-azapentan und 1,3,5-Triamino-n-hexan.

Die 4-Oxopiperidine und die primären Amine können in stöchiometrischen Mengen eingesetzt werden. Von Fall zu Fall kann ein Überschuß oder Unterschuß des 4-Oxopiperidins, z.B. von 0,5 Mol, zweckmäßig von 0,01 bis 0,4 Mol je Äquivalent primärer Aminogruppen, empfehlenswert sein.

Zu den Hydrierbedingungen zählt die Anwesenheit eines geeigneten Hydrierkatalysators. Als solche werden fest angeordnete Trägerkatalysatoren mit einem oder mehreren Metallen der VIII. Nebengruppe des Periodensystems verwendet. Geeignete Träger sind alle inerten Materialien, die die herrschenden Drücke aushalten. Bevorzugt werden Träger aus Aluminiumoxid, Siliciumdioxid und/oder Silikaten. Bevorzugte Edelmetalle aus der VIII. Nebengruppe sind die Platinmetalle Ruthenium, Palladium und Platin, bevorzugte Nichtedelmetalle Cobalt und Nickel. Der Anteil der ersteren in den Trägerkatalysatoren beträgt bis zu 20 Gew.-%, vorteilhaft bis zu 10 Gew.-% und insbesondere 0,5 bis 5 Gew.-%. Der Anteil der Nichtedelmetalle liegt im allgemeinen im Bereich bis zu 40 Gew.-%, vorteilhaft bis zu 20 Gew.-% und insbesondere 3 bis 15 Gew.-%. Die Metalle können vor der eigentlichen reduktiven Aminierungsreaktion durch Reduktion der entsprechenden Oxide oder anderer geeigneter Verbindungen mittels Wasserstoff hergestellt werden. Alternativ kann man diese Reduktion mit der reduktiven Aminierung verbinden. Vorzugsweise stellt man die Katalysatoren so her, daß die Metalle relativ grobteilig vorliegen und nicht pyrophor sind. Dem Katalysatorfachmann sind hierzu geeignete Methoden bekannt.

Man kann einen oder ein Gemisch aus mehreren verschiedenen Trägerkatalysatoren für eine einzige Katalysatorschicht verwenden. Alternativ kann man die Trägerkatalysatoren in mehreren Schichten mit gleichen oder unterschiedlichen Metallen in gleicher oder unterschiedlicher Konzentration einsetzen. Dabei kann ein Konzentrationsgradient bestehen, z.B. ein ansteigender vom Beginn der Reaktionszone bis zu deren Ende. Die Konzentration kann aber auch in Strömungsrichtung abnehmen. Das kann z.B. bei gleichzeitiger Verwendung von Edelmetallen und Nichtedelmetallen als Katalysator nützlich sein. Statt mehrerer Schichten Trägerkatalysator kann man natürlich auch mehrere hintereinandergeschaltete Reaktoren einsetzen.

Es ist ein Vorteil des Verfahrens nach der Erfindung, daß es ohne Lösemittel durchgeführt wird.

Die reduktive Aminierung nach der Erfindung wird im allgemeinen bei Wasserstoffpartialdrücken von 1 bis 500 bar, zweckmäßig von 20 bis 300 bar durchgeführt. Die Reaktionstemperaturen liegen bei 80 bis 200°C, insbesondere bei 90 bis 190°C. Die LHSV sind überraschend hoch und betragen, wie bereits erwähnt, bis zu 1 h⁻¹.

Das Verfahren nach der Erfindung kann z.B. als Rieselverfahren durchgeführt werden, bei dem man die unter den Verfahrensbedingungen flüssigen Ausgangsstoffe über den Trägerkatalysator rieseln läßt, während der Wasserstoff an eineroderan mehreren Stellen der Reaktionszone eingebracht und im Gleichstrom oder im Gegenstrom geführt wird.

Das Reaktionsgemisch wird zweckmäßig durch Destillation untervermindertem Druck gereinigt. Die Destillate entsprechen, wie bereits gesagt, den üblichen hohen Anforderungen an Reinheit und Lagerstabilität.

Die folgenden Beispiele sollen das Verfahren nach der Erfindung weiter erläutern, nicht aber deren Anwendungsbereich begrenzen, wie er aus den Patentansprüchen ersichtlich ist. Alle Prozentangaben beziehen sich auf das Gewicht, soweit nicht anders angegeben.

### Beispiel 1

Ein 500 ml Rieselreaktorwurde mit 400 ml eines Trägerkatalysators (0,5 Gew.-% Palladium auf Aluminiumoxid) beschickt. 2,2,6,6-Tetramethyl-4-oxopiperidin (TAA; Triacetonamin) und Hexamethylendiamin (HDA) im Volumenverhältnis von 2,44:1, entsprechend einem Molverhältnis von 2:1, wurden unter einem Wasserstoffdruck von 285 bar und einer Temperatur von 110°C über den Katalysator geführt. Die LHSV (TAA + HDA) betrug 0,65 h⁻¹. Aus 97 %-igem TAA (GC-Analyse) entstand ein 94 %-iges N,N'-Bis(2,2,6,6-tetramethyl-4-piperidyl)-1,6-hexandiamin, aus dem durch Destillation ein Produkt mit einer Reinheit >99% mit hoher Lagerstabilität gewonnen wurde.

### Beispiel 2

Ein 0,5 m³ Rieselreaktor wurde mit 400 l eines Trägerkatalysators (0,5 Gew.-% Palladium auf Aluminiumoxid) beschickt. 2,2,6,6-Tetramethyl-4-oxopiperidin (TAA) und Hexamethylendiamin (HDA) im Volumenverhältnis von 2,44: 1, entsprechend einem Molverhältnis von 2:1, wurden unter einem Wasserstoffdruck von 285 bar und einer Temperatur von 110°C über den Katalysator geführt. Die LHSV (TAA + HDA) betrug 0.60 h⁻¹. Aus 97 %-igem TAA (GC-Analyse) entstand ein 94.5 %-iges N,N'-Bis(2,2,6,6-tetramethyl-4-piperidyl)-1,6-hexandiamin, aus dem durch Destillation ein Produkt mit einer Reinheit >99% mit hoher Lagerstabilität gewonnen wurde.

### Beispiel 3

Ein 500 ml Rieselreaktorwurdewie im Beispiel 1 mit Trägerkatalysator beschickt. TAA und n-Butylamin (NBA) im Volumenverhältnis von 1,55:1, entsprechend 1 Mol TAA : 1,1 Mol NBA, wurden unter einem Wasserstoffdruck von 285 bar bei einer Temperatur von 110°C über den Katalysator geführt. Die LHSV (TAA + NBA) betrug 0,7 h⁻¹. Aus 97 %-igem TAA (GC-Analyse) wurde als rohes Reaktionsprodukt ein 95iges N-Butyl-2,2,6,6-tetramethyl-4-piperidylamin gewonnen, das durch Destillation unter vermindertem Druck gereinigt wurde.

### Beispiel 4

Ein 500 ml Rieselreaktor wurde wie im Beispiel 1 mit Trägerkatalysator beschickt. TAA und 3-Methoxypropylamin (MPA) im Volumenverhältnis von 1,59:1, entsprechend 1 Mol TAA : 1,05 Mol MPA, wurden unter einem Wasserstoffdruck von 285 bar bei einer Temperatur von 110°C über den Katalysator geführt. Die LHSV (TAA + MPA) betrug 0,75 h⁻¹. Aus 97 %-igem TAA (GC-Analyse) wurde als rohes Reaktionsprodukt ein 94%-iges N-(3-Methoxypropyl)-2,2,6,6-tetramethyl-4-piperidylamin gewonnen, das durch Destillation unter vermindertem Druck gereinigt wurde.

### Beispiel 5

Ein 500 ml Rieselreaktor wurde wie im Beispiel 1 mit Trägerkatalysator beschickt. TAA und 3-Amino-1-propanol (3-AP) im Volumenverhältnis von 2,1:1, entsprechend 1 Mol TAA : 1,05 Mol 3-AP. wurden unter einem Wasserstoffdruck von 285 bar bei einer Temperatur von 110°C über den Katalysator geführt. Die LHSV (TAA + 3-AP) betrug 0,9 h⁻¹. Aus 97 %-igem TAA (GC-Analyse) wurde als rohes Reaktionsprodukt ein 95-iges N-(3-Hydroxypropyl)-2,2,6,6-tetramethyl-4-piperidylamin gewonnen, das durch Destillation unter vermindertem Druck gereinigt wurde.

### Beispiel 6

Ein 500 ml Rieselreaktor wurde wie im Beispiel 1 mit Trägerkatalysator beschickt. TAA und 2-Ethylaminoethylamin (EAEA) im Volumenverhältnis von 1,54:1, entsprechend 1 Mol TAA : 1,05 Mol EAEA, wurden unter einem Wasserstoffdruck von 285 bar bei einer Temperatur von 110°C über den Katalysator geführt. Die LHSV (TAA + EAEA) betrug 0,75 h⁻¹. Aus 97 %-igem TAA (GC-Analyse) wurde als rohes Reaktionsprodukt ein 94-iges N-(2-Ethylaminoethyl)-2,2,6,6-tetramethyl-4-piperidylamin gewonnen, das durch.Destillation unter vermindertem Druck gereinigt wurde.

### Beispiel 7

Ein 500 ml Rieselreaktor wurde wie im Beispiel 1 mit Trägerkatalysator beschickt. TAA und Cyclohexylamin (CHA) im Volumenverhaltnis von 1,42:1, entsprechend 1 Mol TAA : 1,05 Mol CHA, wurden unter einem Wasserstoffdruck von 285 bar bei einer Temperatur von 110°C über den Katalysator geführt. Die LHSV (TAA + CHA) betrug 0,8 h⁻¹. Aus 97 %-igem TAA (GC-Analyse) wurde als rohes Reaktionsprodukt ein 93%-iges N-Cyclohexyl-2,2,6,6-tetramethyl-4-piperidylamin gewonnen, das durch Destillation unter vermindertem Druck weiter gereinigt wurde.

### Beispiel 8

Ein 500 ml Rieselreaktor wurde wie im Beispiel 1 mit Trägerkatalysator beschickt, der 1 Gew.-% Ruthenium auf Al₂O₃ als Träger enthielt. TAA und 3-Amino-1-propanol (3-AP) im Volumenverhältnis von 2,1:1, entsprechend 1 Mol TAA : 1,05 Mol 3-AP, wurden unter einem Wasserstoffdruck von 285 bar bei einer Temperatur von 100°C über den Katalysator geführt. Die LHSV (TAA + 3-AP) betrug 0,6 h⁻¹. Aus 97 %-igem TAA (GC-Analyse) wurde als rohes Reaktionsprodukt ein 93%-iges N-(3-Hydroxypropyl)-2,2,6,6-tetramethyl-4-piperidylamin gewonnen, das durch Destillation unter vermindertem Druck weiter gereinigt wurde.

## Patentansprüche

1. Verfahren zur Herstellung von N-monosubstituierten 4-Aminopiperidinen durch kontinuierliche, einstufige Umsetzung eines 4-Oxopiperidins mit einem primären Amin und Wasserstoff unter Hydrierungsbedingungen,
**dadurch gekennzeichnet,**
**daß** das 4-Oxopiperidin der allgemeinen Formel 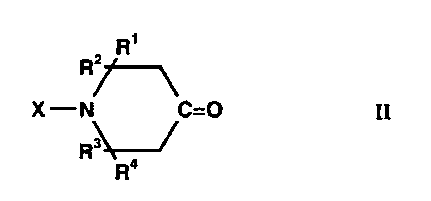 entspricht, in der
- R¹, R², R³ und R⁴ gleich oder verschieden sein können und Wasserstoff oder einen Kohlenwasserstoffrest mit 1 bis 4 Kohlenstoffatomen bedeuten;
- X für Wasserstoff, eine Hydroxylgruppe, einen Kohlenwasserstoffrest mit 1 bis 18 Kohlenstoffatomen, einen Acylrest mit 2 bis 10 Kohlenstoffatomen, für eine gegebenenfalls N,N-dialkylierte Amino- oder Aminoalkylgruppe mit bis zu 12 Kohlenstoffatomen oder für einen endständig substituierten Alkylenrest der Formel
- (CH₂)ₘ-R⁵ steht, in der R⁵ eine Hydroxylgruppe oder einen Alkoxyrest mit 1 bis 6 Kohlenstoffatomen und m eine ganze Zahl von 1 bis 6 bedeutet,
und das primäre Amin einer der allgemeinen Formeln
Y-NH₂ III
oder
Z-(NH₂)ₙ V
entspricht, in denen
- Y einen einwertigen Kohlenwasserstoffrest mit 1 bis 12 Kohlenstoffatomen bedeutet, der gegebenenfalls Etherbrücken, (N-Alkyl)iminobrücken und/oder Hydroxylgruppen enthält und/oder arylsubstituiert ist.
- Z einen n-wertigen Kohlenwasserstoffrest mit 2 bis 12 Kohlenstoffatomen bedeutet der gegebenenfalls Etherbrücken, (N-Alkyl)iminobrücken und/oder Hydroxylgruppen enthält und/oder arylsubstituiert ist, und
- n für eine ganze Zahl von 2 bis 4 steht
und wobei man die Edukte über einen fest angeordneten Trägerkatalysator führt, der ein oder mehrere Metalle der VIII. Nebengruppe des Periodensystems enthält und man die Reaktion ohne Lösemitteleinsatz bei 80 bis 200 °C durchführt.

2. Verfahren nach Anspruch 1,
**dadurch gekennzeichnet,**
**dass** der Trägerkatalysator Kobalt, Nickel, Ruthenium, Palladium und/oder Platin enthält.

3. Verfahren nach einem der Ansprüche 1 bis 2,
**dadurch gekennzeichnet,**
**dass** ein vom Beginn bis zum Ende der Reaktionszone ansteigender Konzentrationsgradient des Metalls oder der Metalle besteht.

4. Verfahren nach einem der Ansprüche 1 bis 3,
**dadurch gekennzeichnet,**
**dass** man die Reaktion bei 90 bis 190 °C durchführt.

## Claims

1. A process for preparing an N-monosubstituted 4-aminopiperidine by continuous single-stage reaction of a 4-oxopiperidine with a primary amine and hydrogen under hydrogenation conditions,
**characterized in that** the 4-oxopiperidine corresponds to the general formula 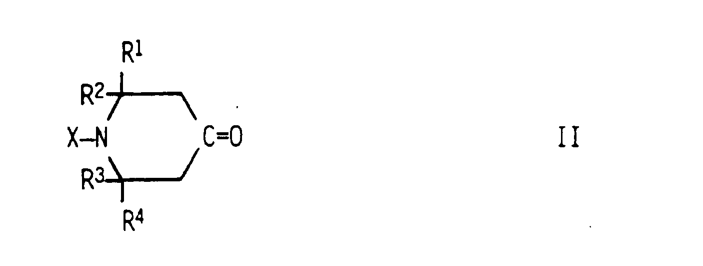 in which
- R¹, R², R³ and R⁴ can be identical or different and denote hydrogen or a hydrocarbon radical having from 1 to 4 carbon atoms;
- X represents hydrogen, a hydroxyl group, a hydrocarbon radical having from 1 to 18 carbon atoms, an acyl radical having from 2 to 10 carbon atoms, an amino or aminoalkyl group having up to 12 carbon atoms which, if desired, is N,N-dialkylated, or a terminally substituted alkylene radical of the formula -(CH₂)ₘ-R⁵, in which R5 denotes a hydroxyl group or an alkoxy radical having from 1 to 6 carbon atoms and m denotes an integer from 1 to 6,
and the primary amine corresponds to one of the general formulae
Y-NH₂ III
or
Z-(NH₂)ₙ V,
in which
- Y denotes a monovalent hydrocarbon radical having from 1 to 12 carbon atoms which, if desired, contains ether bridges, (N-alkyl)imino bridges and/or hydroxyl groups and/or is aryl substituted,
- Z denotes an n-valent hydrocarbon radical having from 2 to 12 carbon atoms, which, if desired, contains ether bridges, (N-alkyl) imino bridges and/or hydroxyl groups and/or is aryl substituted, and
- n represents an integer from 2 to 4,
and where the starting materials are conducted over a fixed supported catalyst which contains one or more metals of subgroup VIII of the Periodic Table of the Elements and the reaction is carried out at from 80 to 200°C without the use of solvent.

2. A process according to claim 1, **characterized in that** the supported catalyst contains cobalt, nickel, ruthenium, palladium and/or platinum.

3. A process according to either one of claims 1 and 2, **characterized in that** there is a concentration gradient of the metal or the metals which increases from the start to the end of the reaction zone.

4. A process according to any one of claims 1 to 3, **characterized in that** the reaction is carried out at from 90 to 190°C.

## Revendications

1. Procédé d'obtention de 4-aminopipéridines N-monosubstituées par réaction en continu en une seule étape, d'une 4-oxopipéridine avec une amine primaire et de l'hydrogène dans des conditions d'hydrogénation,
**caractérisé en ce que**
la 4-oxopipéridine correspond à la formule générale : 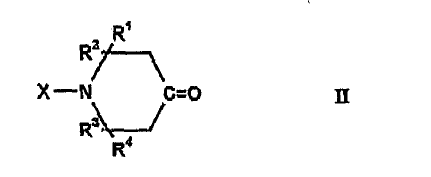 dans laquelle
- R¹, R², R³ et R⁴ peuvent être identiques ou différents, et signifient de l'hydrogène ou un reste hydrocarboné ayant de 1 à 4 atomes de carbone,
- X représente de l'hydrogène, un groupe hydroxyle, un reste hydrocarboné ayant de 1 à 18 atomes de carbone, un reste acyle ayant de 2 à 10 atomes de carbone, un groupe amino ou aminoalkyle éventuellement N,N-dialkylé ayant jusqu'à 12 atomes de carbone ou un reste alkylène substitué terminal, de formule -(CH₂)ₘ-R⁵ dans laquelle R⁵ signifie un groupe hydroxyle ou un reste alkoxy ayant de 1 à 6 atomes de carbone, et m un nombre entier allant de 1 à 6,
et l'amine primaire correspond à une des formules générales :
Y-NH₂ III
ou
Z-(NH₂)ₙ V
dans lesquelles
- Y signifie un reste hydrocarboné monofonctionnel ayant de 1 à 12 atomes de carbone, qui contient éventuellement des ponts éther, des ponts (N-alkyl)imino et/ou des groupes hydroxyle et/ou est substitué par un aryle,
- Z signifie un reste hydrocarboné n-fonctionnel ayant de 2 à 12 atomes de carbone qui contient éventuellement des ponts éther, des ponts (N-alkyl)imino et/ou des groupes hydroxyle et/ou est substitué par un aryle, et
- n représente un nombre entier allant de 2 à 4,
et on amène les éduits sur un catalyseur sur support disposé solidement, qui renferme un ou plusieurs métaux du VIIIe sous-groupe du système périodique, et on effectue la réaction sans mise en oeuvre de solvant entre 80°C et 200°C.

2. Procédé selon la revendication 1,
**caractérisé en ce que**
le catalyseur sur support renferme du cobalt, du nickel, du ruthénium, du palladium et/ ou du platine.

3. Procédé selon l'une quelconque des revendications 1 ou 2,
**caractérisé par**
un gradient de concentration du métal ou des métaux croissant du début jusqu'à la fin de la zone de réaction.

4. Procédé selon l'une quelconque des revendications 1 à 3,
**caractérisé en ce qu'**
on effectue la réaction de 90°C à 190°C.
